# EUROPÄISCHE PATENTANMELDUNG

(11) **EP 4 818 153 A1**
(43) Veröffentlichungstag der Anmeldung: **07.10.2026**
(21) Anmeldenummer: 25167739.9
(22) Anmeldetag: 01.04.2025
(51) Int. Cl.: C07C 253/10, C07C 255/46

(54) **VERFAHREN ZUR HERSTELLUNG VON ISOPHORONNITRIL**

(71) Anmelder: Evonik Operations GmbH, 45128 Essen (DE)
(72) Erfinder: METTERNICH, Jan Benedikt, 45657 Recklinghausen (DE); NITZ, Jörg-Joachim, 45257 Essen (DE); REITMEYER, Michael, 59399 Olfen (DE); MENDORF, Matthias, 44225 Dortmund (DE); SOBOLL, Sebastian, 45665 Recklinghausen (DE); JOHN, Christian, 44625 Herne (DE); ALBERS, Katja, 46147 Oberhausen (DE); BAHLMANN, Matthias, 46325 Borken (DE); SPEYERER, Christian, 44809 Bochum (DE); KOHLSTRUK, Stephan, 45966 Gladbeck (DE)
(74) Vertreter: Evonik Patent Association

(57) **Zusammenfassung**

Gegenstand der vorliegenden Erfindung ist ein Verfahren zur Herstellung von Isophoronnitril durch Umsetzung von Isophoron mit Cyanwasserstoff in Gegenwart einer Base und nachfolgende Neutralisation, bei dem die Neutralisation mit Zitronensäure erfolgt.

## Beschreibung

Der Gegenstand der vorliegenden Erfindung ist ein Verfahren zur Herstellung von Isophoronnitril.

Isophoronnitril ist eine bedeutende Industriechemikalie, deren Relevanz unter anderem darauf beruht, dass sie als Edukt für die Synthese von als Epoxyhärter eingesetztem Isophorondiamin und des daraus herstellbaren Isophorondiisocyanats verwendet wird, das von großer Relevanz für die Polyurethanchemie ist. Aus diesem Grund sind Verfahren zur Herstellung von Isophoronnitril von großem Interesse. Üblicherweise wird Isophoronnitril aus Isophoron hergestellt.

Im Stand der Technik sind Verfahren zur Herstellung von Isophoronnitril beschrieben: EP 425 806 A1 offenbart ein Verfahren zur Herstellung von Isophoronnitril, bei dem Isophoron mit Alkalicyanid umgesetzt und zur Gewährleistung eines pH-Wertes zwischen 8 und 14 eine anorganische Säure zugesetzt wird. Bevorzugt wird als anorganische Säure Phosphorsäure oder Schwefelsäure zugegeben.

Im Stand der Technik haben sich jedoch aus Gründen des verbesserten Handlings Synthesen durchgesetzt, die anstelle von Alkalicyanid Cyanwasserstoff (HCN) einsetzen.

EP 433 615 A1 offenbart ein Verfahren zur Herstellung von Isophoronnitril, bei dem Cyanwasserstoff in Gegenwart einer alkalisch wirkenden Alkaliverbindung als Katalysator an Isophoron angelagert wird. Die Alkaliverbindung ist Lithiumhydroxid. Die Aufarbeitung kann eine Neutralisation oder Abtrennung des Katalysators vorsehen. Die Neutralisation der Alkaliverbindung kann mit Phosphorsäure oder p-Toluolsulfonsäure erfolgen.

US 5,011,968 A1 offenbart ein Verfahren zur Herstellung von Isophoronnitril aus Isophoron und HCN in Gegenwart eines quaternären Ammoniumhydroxid-Katalysators. Die Neutralisation des Katalysators kann mit einer Säure, insbesondere Phosphorsäure, Salpetersäure oder Schwefelsäure erfolgen.

Bei den in EP 433 615 A1 und US 5,011,968 A1 offenbarten Säuren handelt es sich vor allem um anorganische Säuren, die nachteilig sind, weil verbleibende Rückstände der anorganischen Säuren im Isophoronnitril-Rohprodukt die Lagerstabilität verringern. Die vollständige Abtrennung der Säuren aus dem Rohprodukt ist aufwändig. Zusätzlich sind die eingesetzten anorganischen Säuren korrosiv und erhöhen den Aufwand für den Erhalt der Produktionsanlagen. Zuletzt stellen oxidierende Säuren ein signifikantes Sicherheitsrisiko dar und erhöhen den sicherheitstechnischen Aufwand, um eine sichere Nutzung zu ermöglichen.

CN 116178213 A offenbart ein Verfahren zur Herstellung von Isophoronnitril aus Isophoron und Cyanwasserstoff in Gegenwart eines alkalischen Katalysators, bei dem eine Säure für die Neutralisation verwendet wird. Bevorzugt wird die Säure ausgewählt aus der Gruppe der organischen Sulfonsäuren.

Organische Sulfonsäuren weisen jedoch auch Nachteile auf, insbesondere die hohe Löslichkeit organischer Sulfonsäuren in Isophoronnitril. Verbleibende Rückstände der organischen Sulfonsäuren im Isophoronnitril-Rohprodukt verringern die Lagerstabilität und machen somit eine Abtrennung aus dem Rohprodukt notwendig. Die Abtrennung von organischen Sulfonsäure aus Isophoronnitril-Rohprodukt ist mit hohem apparativen und energetischem Aufwand verbunden. Aus diesem Grund wurden Verfahren entwickelt, die organische Säuren einsetzen.

EP 558 332 A1 offenbart ein Verfahren zur Herstellung von Isophoronnitril aus Isophoron und Cyanwasserstoff in Gegenwart eines Katalysators, bei dem eine mehrprotonige Säure für die Neutralisation eingesetzt wird. Als einsetzbare organische mehrprotonige Säuren werden Maleinsäure und Oxalsäure genannt.

Auch CN 104230756 A offenbart ein Verfahren zur Herstellung von Isophoronnitril aus Isophoron und Cyanwasserstoff in Gegenwart eines Katalysators, bei dem eine Säure für die Neutralisation eingesetzt wird. Als organische mehrprotonige Säuren werden Maleinsäure, Phthalsäure und Oxalsäure genannt.

Die in EP 558 332 A1 und CN 104230756 A genannten organischen mehrprotonigen Säuren sind Disäuren. Nach Verwendung organischer Disäuren resultieren jedoch in der organischen Phase zu hohe Natriumgehalte, zu hohe pH-Werte und zu hohe Restsäuregehalte.

WO 2004/056753 A1 offenbart ein Verfahren zur Herstellung von Isophoronnitril aus Isophoron und Cyanwasserstoff in Gegenwart eines Katalysators, bei dem eine Sulfon- oder Carbonsäure eingesetzt werden kann. Als einsetzbare Carbonsäuren sind auch aliphatische Polycarbonsäuren mit 3 oder mehr, bevorzugt 3 - 100 Carboxylgruppen offenbart. Bevorzugt einsetzbare Polycarbonsäuren werden jedoch nicht offenbart.

Gegenüber dem aufgeführten Stand der Technik stellt sich die Aufgabe, ein Verfahren zur Herstellung von Isophoronnitril aus Isophoron und Cyanwasserstoff bereitzustellen, mit dem nach Neutralisation ein Isophoronnitril mit besonders niedrigem Alkali- und Erdalkalimetallgehalt, besonders niedrigem Restsäuregehalt und einem nah an 7 liegendem pH-Wert erhalten werden kann. Hohe Alkalimetall-, Erdalkalimetall- und Restsäuregehalte sind nachteilig, da sie die Lagerstabilität des Isophoronnitrils nachteilig beeinflussen. Nachteilig an hohen Alkalimetall-, Erdalkalimetall- und Restsäuregehalten ist weiterhin, dass sie in der Umsetzung von Isophoronnitril mit Ammoniak zu Isophorondiamin, dem bedeutendsten Einsatzzweck von Isophoronnitril, den Katalysator negativ beeinflussen und zur Spaltung des zwischenzeitlich aus Ammoniak und Isophoronnitril gebildeten Imins führen können. Aus gleichem Grund, d.h. wegen geringer Lagerstabilitäten des Isophoronnitrils, negativer Einflüsse auf den Katalysator und einer erhöhten Spaltung des aus Ammoniak und Isophoronnitril gebildeten Imins bei der Synthese von Isophorondiamin, sind auch stark von pH = 7 abweichende pH-Werte nachteilig.

Überraschenderweise wurde festgestellt, dass die sich vorliegend stellende Aufgabe gelöst wird, wenn Zitronensäure für die Neutralisation eingesetzt wird. Wird Zitronensäure für die Reaktion eingesetzt, kann die Aufarbeitung ggü. einer entsprechenden Reaktion, die stattdessen anorganische Säuren oder organische Sulfonsäuren einsetzt, vereinfacht werden. Weiterhin werden im Vergleich mit anderen organischen Disäuren niedrigere Natriumgehalte und Restsäure-konzentrationen im Isophoronnitril erzielt. Weiterhin liegt der pH-Wert näher an pH = 7. Auch ggü. anderen organischen Trisäuren als Zitronensäure resultieren Vorteile: so wird auch ggü. einer entsprechenden Neutralisation mit Propantricarbonsäure ein niedrigerer Restsäuregehalt und eine geringere Natriumkonzentration im Isophoronnitril erzielt.

Gegenstand der vorliegenden Erfindung ist somit ein Verfahren zur Herstellung von Isophoronnitril durch Umsetzung von Isophoron mit Cyanwasserstoff in Gegenwart einer Base und nachfolgende Neutralisation, bei dem die Neutralisation mit Zitronensäure erfolgt.

Die vorliegende Erfindung ist ein Verfahren zur Herstellung von Isophoronnitril durch Umsetzung von Isophoron mit Cyanwasserstoff in Gegenwart einer Base.

Das eingesetzte Isophoron stammt üblicherweise aus einer basenkatalysierten Aldolkondensation von Aceton zu Isophoron. Isophoron ist im Handel erhältlich. Auch Cyanwasserstoff (Blausäure) ist im Handel erhältlich.

Die Umsetzung von Isophoron mit Cyanwasserstoff wird in Gegenwart einer Base als Katalysator durchgeführt, da die Base freie Cyanid-Ionen für die 1,4-Additionsreaktion generiert.

Bevorzugte Basen können ausgewählt werden aus der Gruppe bestehend aus basischen Alkali- oder Erdalkali-verbindungen, Oniumverbindungen, organischen Basen und Phasentransferkatalysatoren. Ganz besonders bevorzugt kann die Base ausgewählt werden aus der Gruppe bestehend aus basischen Alkali- oder Erdalkalimetallverbindungen. Noch weiter bevorzugt kann die Base ausgewählt werden aus der Gruppe bestehend aus Alkalialkoholaten, -oxiden, -hydroxiden, -carbonaten, -peroxiden und - cyaniden, sowie Erdalkalialkoholaten, -oxiden, -hydroxiden, -peroxiden und -cyaniden.

Die Umsetzung mit Cyanwasserstoff (HCN) kann in Lösung oder lösemittelfrei durchgeführt werden.

Die Basenkatalyse kann homogen oder heterogen durchgeführt werden. Bei der homogenen Katalyse liegen Katalysator und Reaktanden in der gleichen Phase vor. Bei der heterogenen Katalyse liegen Katalysator und Reaktanden in unterschiedlichen Phasen vor.

Bevorzugt wird bei dem erfindungsgemäßen Verfahren eine homogene Basenkatalyse eingesetzt. Hierzu werden ganz besonders bevorzugt Alkalialkoholate, insbesondere Natriummethanolat, als Katalysatoren eingesetzt.

Der Cyanwasserstoff kann in Reinform, aber auch als Gemisch mit Stabilisatoren eingesetzt werden. Als Stabilisatoren eignen sich alle dem Fachmann für diesen Zweck bekannten Verbindungen, beispielsweise Phosphorsäure, Schwefeldioxid und Oxalsäure. Bevorzugt werden Phosphorsäure, Schwefeldioxid oder Gemische hieraus als Stabilisatoren für die Blausäure eingesetzt. Der Anteil der Stabilisatoren liegt üblicherweise im Bereich von 0,1 bis 2 Gew.-%, bezogen auf die eingesetzte Blausäure, vorzugsweise im Bereich von 0,5 bis 1 Gew.-%.

Die Umsetzung kann in Gegenwart oder Abwesenheit von inerten Lösungsmitteln vorgenommen werden. Besonders bevorzugt verwendet man Isophoron im molaren Überschuss, bezogen auf die eingesetzte Blausäure und setzt kein externes Lösungsmittel zu.

Bevorzugt wird das erfindungsgemäße Verfahren so durchgeführt, dass ein Überschuss an Isophoron eingesetzt wird, da so eine höhere Selektivität zu Isophoronnitril erzielt wird. Bevorzugt beträgt das molare Verhältnis Isophoron zu Cyanwasserstoff (HCN) > 1 : 1, weiter bevorzugt 19 : 1 bis 1,5 : 1, vorzugsweise 3 : 1 bis 1,5 : 1.

Die Basenkonzentration, bezogen auf die eingesetzte Isophoronmenge, liegt im Bereich von 0,03 bis 20 Gew.-%, vorzugsweise im Bereich von 0,03 bis 1 Gew.-%.

Bevorzugt wird die Gesamtmenge des Isophorons vorgelegt und auf die gewünschte Reaktionstemperatur gebracht, bevor der Cyanwasserstoff in Anwesenheit der Base zugegeben wird.

Die HCN-Zudosierung erfolgt vorzugsweise so, dass eine homogene Verteilung der Edukte (Isophoron und HCN) gewährleistet wird. Die HCN-Zudosierung kann auf übliche, dem Fachmann bekannte Weise erfolgen, bevorzugt über Statikmischer, Pumpen oder Verdüsung.

Die Reaktionstemperaturen liegen üblicherweise im Bereich von 130 bis 225 °C, bevorzugt im Bereich von 135 bis 195 °C und ganz besonders bevorzugt im Bereich von 140 bis 175 °C.

Die Reaktion wird üblicherweise bei Drücken von 0,01 bis 10 bar, bevorzugt 1 bis 5 bar, besonders bevorzugt bei Normaldruck (Atmosphärendruck) durchgeführt.

Innerhalb der vorstehend dargelegten Reaktionsparameter wird das HCN bevorzugt so zudosiert, dass eine ausreichend niedrige Konzentration von HCN resultiert und somit eine hohe Selektivität sowie ein hoher Umsatz zu Isophoronnitril erzielt werden kann.

Vorzugsweise werden die Konzentrationen an nicht umgesetztem, freiem HCN und die Gesamtkonzentration an Cyanidionen (Summe aus freiem HCN und als Cyanhydrine von Isophoron und Isophoronnitril gebundenem Cyanid) bestimmt und die Reaktionsbedingungen so angepasst, dass eine optimierte Selektivität hinsichtlich Isophoronnitril erhalten wird. Die Bestimmung der genannten Cyanidionen-Konzentrationen erfolgt vorzugsweise durch Titration.

Das erfindungsgemäße Verfahren kann in bevorzugter Weise kontinuierlich, diskontinuierlich oder semikontinuierlich durchgeführt werden, insbesondere kontinuierlich.

In bevorzugter Ausführung wird die Reaktionsdurchführung, je nach eingesetztem Katalysator, in einem Rührkessel, einer Rührkesselkaskade, einem Kreislaufreaktor, einem Strömungsrohr, einem oder mehreren Festbettreaktoren oder einer Kolonne durchgeführt.

Vorzugsweise wird überschüssiges Isophoron durch Destillation abgetrennt und vorteilhafterweise erneut eingesetzt. Dazu wird das abgetrennte Isophoron mit frischem Isophoron vermischt und in den Reaktionsreaktor zurückgeführt. Das gebildete Isophoronnitril wird durch destillative Aufarbeitung von möglichen Nebenkomponenten abgetrennt und kann weiter eingesetzt werden.

Nach der Umsetzung von Isophoron mit Cyanwasserstoff in Gegenwart einer Base erfolgt eine Neutralisation mit Zitronensäure.

Bevorzugt erfolgt die Neutralisation mit Zitronensäure durch die Zugabe einer wässrigen Zitronensäure-Lösung. Dies hat den Vorteil, dass nach der Neutralisation zwei Phasen vorliegen, und zwar eine organische Isophoronnitril-reiche Lösung, die ggf. auch den Hauptanteil organischer Nebenprodukte sowie ggf. nicht umgesetztes Isophoron enthält, und eine wässrige, Alkalimetall- und Erdalkalimetall- und citratreiche Lösung, die sich leicht abtrennen lässt. Weiter bevorzugt erfolgt somit nach der Neutralisation eine Separation der entstehenden Phasen mit erhöhtem Natriumgehalt in der wässrigen Phase.

Bevorzugt beträgt die Zitronensäurekonzentration in der wässrigen Lösung 0,1 - 10 Gew.-%, weiter bevorzugt 0,5 - 2 Gew.-%, noch weiter bevorzugt 0,5 - 1 Gew.-%, bezogen auf die eingesetzte Masse an Wasser.

Das Massenverhältnis aus eingesetztem Roh-Isophoronnitril (d.h. dem direkten Produkt der Umsetzung von Isophoron und Cyanwasserstoff in Gegenwart einer Base) und wässriger Zitronensäure-Lösung beträgt bevorzugt 1:1 - 25:1, weiter bevorzugt 5:1 - 20:1, ganz besonders bevorzugt 10:1 - 15:1.

Bevorzugt wird die Neutralisation bei einer Temperatur von 20 - 90 °C, weiter bevorzugt 30 - 80 °C, noch weiter bevorzugt 40 - 70 °C durchgeführt.

Erfolgen Umsetzung und Neutralisation kontinuierlich, wird die Neutralisation bevorzugt in einer Extraktionskolonne durchgeführt. Dabei wird die Extraktionskolonne bevorzugt voll-kontinuierlich betrieben.

Weiter bevorzugt erfolgt die Neutralisation im Gegenstrombetrieb unter Zufuhr des Roh-Isophoronnitrils am Boden der Kolonne und unter Zufuhr der wässrigen Zitronensäurelösung am Kopf der Kolonne.

Bevorzugt wird in der Kolonne eine Füllung bestehend aus einer Metallpackung oder Keramikfüllkörpern eingesetzt.

Besonders bevorzugt erfolgt nicht nur die Neutralisation nach der Umsetzung, sondern auch die Separation der entstehenden Phasen in der Extraktionskolonne. Dabei kann die organische Isophoronnitril-reiche Lösung am Kopf der Kolonne und die wässrige, Alkalimetall-, Erdalkalimetall-, bevorzugt Natrium- und citratreiche Lösung am Boden der Kolonne entnommen werden.

Die aus der Separation stammende organische Isophoronnitril-reiche Lösung wird bevorzugt zur weiteren Aufreinigung, insbesondere zur Entfernung verbliebener Citrat-Spuren wässrig extrahiert. Weiter bevorzugt wird die wässrige Extraktion in einer weiteren Extraktionskolonne durchgeführt. Weiter bevorzugt wird diese weitere Extraktionskolonne voll-kontinuierlich betrieben.

Weiter bevorzugt erfolgt die Extraktion im Gegenstrombetrieb unter Zufuhr der aus der Separation stammenden organischen Isophoronnitril-reichen Lösung am Boden der Kolonne und unter Zufuhr von Wasser am Kopf der Kolonne.

Bevorzugt wird in dieser Kolonne eine Füllung bestehend aus einer Metallpackung oder Keramikfüllkörpern eingesetzt.

Das Massenverhältnis von aus der Separation stammender organischer Isophoronnitril-reicher Lösung und Wasser beträgt bevorzugt 1:1 - 25:1, weiter bevorzugt 5:1 - 20:1, ganz besonders bevorzugt 10:1 - 15:1.

Bevorzugt erfolgt die wässrige Extraktion bei 20 - 90 °C, weiter bevorzugt 30 - 80 °C, noch weiter bevorzugt 40 - 70 °C.

Bevorzugt kann das mit dem Verfahren erhältliche Isophoronnitril eingesetzt werden für die Synthese von Isophorondiamin über aminierende Hydrierung. Gegenstand der vorliegenden Erfindung ist somit auch die Verwendung des nach dem erfindungsmäßen Verfahrens erhältlichen Isophoronnitrils für die Synthese von Isophorondiamin.

Alle angegebenen Prozent (%) sind, wenn nicht anders angegeben, Massenprozent.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### 1. Batch-Neutralisation von Roh-Isophoronnitril in einem Rührkessel bei 60 °C mit Zitronensäure

Die Batch-Neutralisation wurde in einem 1 L Doppelwand-Behälter mit Propellerrührer umgesetzt. Die Beheizung des Behälters erfolgte via Thermostat mit Wasser. Die eingesetzte Roh-Isophoronnitrilmischung wies folgende Zusammensetzung auf: 52 Gew.-% Isophoron, 44 Gew.-% Isophoron-nitril, 0.06 Gew.-% Natriummethoxid. 600 g des Roh-Isophoronnitrils und 60 g der wässrigen Zitronensäure-Lösung (0.9 Gew.-% Zitronensäure) wurden in dem 1 L Reaktor vorgelegt und auf 60 °C erhitzt. Die Rührzeit betrug 1 h bei 60 °C. Die behandelte Roh-Isophoronnitrilmischung wies nach der Neutralisation einen pH-Wert von pH 5.7, eine Säurezahl von 0.2 mg KOH/g Probe, eine Natriumkonzentration von <5 ppm und eine Zitronensäurekonzentration von 0.0005 Gew.-% auf.

### 2. Batch-Neutralisation von Roh-Isophoronnitril in einem Rührkessel bei 60 °C mit Salpetersäure

Die Batch-Neutralisation wurde in einem 1 L Doppelwand-Behälter mit Propellerrührer umgesetzt. Die Beheizung des Behälters erfolgte via Thermostat mit Wasser. Die eingesetzte Roh-Isophoronnitrilmischung wies folgende Zusammensetzung auf: 52 Gew.-% Isophoron, 44 Gew.-% Isophoron-nitril, 0.06 Gew.-% Natriummethoxid. 600 g des Roh-Isophoronnitrils und 60 g der wässrigen Salpetersäure-Lösung (0.9 Gew.-% Salpetersäure) wurden in dem 1 L Reaktor vorgelegt und auf 60 °C erhitzt. Die Rührzeit betrug 1 h bei 60 °C. Die behandelte Roh-Isophoronnitrilmischung wies nach der Neutralisation einen pH-Wert von pH 2.5, eine Säurezahl von 0.4 mg KOH/g Probe, eine Natriumkonzentration von 13 ppm auf.

### 3. Batch-Neutralisation von Roh-Isophoronnitril in einem Rührkessel bei 60 °C mit Oxalsäure

Die Batch-Neutralisation wurde in einem 1 L Doppelwand-Behälter mit Propellerrührer umgesetzt. Die Beheizung des Behälters erfolgte via Thermostat mit Wasser. Die eingesetzte Roh-Isophoronnitrilmischung wies folgende Zusammensetzung auf: 52 Gew.-% Isophoron, 44 Gew.-% Isophoron-nitril, 0.06 Gew.-% Natriummethoxid. 600 g des Roh-Isophoronnitrils und 60 g der wässrigen Oxalsäure-Lösung (0.9 Gew.-% Oxalsäure) wurden in dem 1 L Reaktor vorgelegt und auf 60 °C erhitzt. Die Rührzeit betrug 1 h bei 60 °C. Die behandelte Roh-Isophoronnitrilmischung wies nach der Neutralisation einen pH-Wert von pH 3.3, eine Säurezahl von 1.2 mg KOH/g Probe, eine Natriumkonzentration von 8 ppm und eine Oxalsäurekonzentration von 0.01 Gew.-% auf.

### 4. Batch-Neutralisation von Roh-Isophoronnitril in einem Rührkessel bei 60 °C mit Phthalsäure

Die Batch-Neutralisation wurde in einem 1 L Doppelwand-Behälter mit Propellerrührer umgesetzt. Die Beheizung des Behälters erfolgte via Thermostat mit Wasser. Die eingesetzte Roh-Isophoronnitrilmischung wies folgende Zusammensetzung auf: 52 Gew.-% Isophoron, 44 Gew.-% Isophoron-nitril, 0.06 Gew.-% Natriummethoxid. 600 g des Roh-Isophoronnitrils und 60 g der wässrigen Phthalsäure-Lösung (0.9 Gew.-% Phthalsäure) wurden in dem 1 L Reaktor vorgelegt und auf 60 °C erhitzt. Die Rührzeit betrug 1 h bei 60 °C. Die behandelte Roh-Isophoronnitrilmischung wies nach der Neutralisation einen pH-Wert von pH 7.4, eine Säurezahl von 0.1 mg KOH/g Probe, eine Natriumkonzentration von 18 ppm und eine Phthalsäurekonzentration von 0.001 Gew.-% auf.

### 5. Batch-Neutralisation von Roh-Isophoronnitril in einem Rührkessel bei 60 °C mit Tricarballylsäure

Die Batch-Neutralisation wurde in einem 1 L Doppelwand-Behälter mit Propellerrührer umgesetzt. Die Beheizung des Behälters erfolgte via Thermostat mit Wasser. Die eingesetzte Roh-Isophoronnitrilmischung wies folgende Zusammensetzung auf: 52 Gew.-% Isophoron, 44 Gew.-% Isophoron-nitril, 0.06 Gew.-% Natriummethoxid. 600 g des Roh-Isophoronnitrils und 60 g der wässrigen Tricarballylsäure-Lösung (0.9 Gew.-% Tricarballylsäure) wurden in dem 1 L Reaktor vorgelegt und auf 60 °C erhitzt. Die Rührzeit betrug 1 h bei 60 °C. Die behandelte Roh-Isophoronnitrilmischung wies nach der Neutralisation einen pH-Wert von pH 5.2, eine Säurezahl von 0.2 mg KOH/g Probe, eine Natriumkonzentration von 8 ppm und eine Tricarballylsäurekonzentration von 0.01 Gew-% auf.

## Patentansprüche

1. Verfahren zur Herstellung von Isophoronnitril durch
- Umsetzung von Isophoron mit Cyanwasserstoff in Gegenwart einer Base und
- nachfolgende Neutralisation,
**dadurch gekennzeichnet,**
**dass** die Neutralisation mit Zitronensäure erfolgt.

2. Verfahren nach Anspruch 1,
**dadurch gekennzeichnet, dass**
die Base ausgewählt wird aus der Gruppe bestehend aus basischen Alkali- oder Erdalkalimetallverbindungen.

3. Verfahren nach Anspruch 2,
**dadurch gekennzeichnet, dass**
die Base ausgewählt wird aus der Gruppe der Alkalialkoholate.

4. Verfahren nach einem der vorhergehenden Ansprüche,
**dadurch gekennzeichnet, dass**
das molare Verhältnis Isophoron zu Cyanwasserstoff > 1 : 1 ist.

5. Verfahren nach einem der vorhergehenden Ansprüche,
**dadurch gekennzeichnet, dass**
die Neutralisation mit Zitronensäure durch Zugabe einer wässrigen Zitronensäure-Lösung erfolgt.

6. Verfahren nach Anspruch 5,
**dadurch gekennzeichnet, dass**
Zitronensäurekonzentration in der wässrigen Lösung 0,1 - 10 Gew.-% bezogen auf die eingesetzte Masse an Wasser beträgt.

7. Verfahren nach Anspruch 5 oder 6,
**dadurch gekennzeichnet, dass**
das Massenverhältnis aus eingesetztem Roh-Isophoronnitril und wässriger Zitronensäure-Lösung 1:1 - 25:1 beträgt.

8. Verfahren nach einem der vorhergehenden Ansprüche,
**dadurch gekennzeichnet, dass**
die Neutralisation bei einer Temperatur von 20 - 90 °C durchgeführt wird.

9. Verfahren nach einem der vorhergehenden Ansprüche,
**dadurch gekennzeichnet, dass**
Umsetzung und Neutralisation kontinuierlich erfolgt und die Neutralisation in einer Extraktionskolonne durchgeführt wird.

10. Verfahren nach Anspruch 9,
**dadurch gekennzeichnet, dass**
die Neutralisation im Gegenstrombetrieb
- unter Zufuhr des Roh-Isophoronnitrils am Boden der Kolonne und
- unter Zufuhr der wässrigen Zitronensäurelösung am Kopf der Kolonne erfolgt.

11. Verfahren nach Anspruch 9 oder 10,
**dadurch gekennzeichnet, dass**
in der Kolonne eine Füllung bestehend aus einer Metallpackung oder Keramikfüllkörpern eingesetzt wird.

12. Verfahren nach einem der Ansprüche 9 bis 11,
**dadurch gekennzeichnet, dass**
auch eine kontinuierlich erfolgende Separation der entstehenden Phasen in der Extraktionskolonne erfolgt.

13. Verfahren nach Anspruch 12,
**dadurch gekennzeichnet, dass**
die aus der Separation stammende organische Isophoronnitril-reiche Lösung wässrig extrahiert wird.

14. Verfahren nach Anspruch 13,
**dadurch gekennzeichnet, dass**
die wässrige Extraktion in einer weiteren Extraktionskolonne durchgeführt wird.

15. Verfahren nach Anspruch 13 oder 14,
**dadurch gekennzeichnet, dass**
die wässrige Extraktion bei 20 - 90 °C erfolgt.
